# EUROPEAN PATENT APPLICATION

(11) **EP 1 380 644 A1**
(43) Date of publication of application: **14.01.2004**
(21) Application number: 02077711.6
(22) Date of filing: 08.07.2002
(51) Int. Cl.: C12N 15/11, C12Q 1/68, C07K 16/18, A61K 39/395, A61K 31/7008, A61K 31/713, A61K 48/00, A61K 31/00, G01N 33/53, A61P 35/00, A61K 38/18

(54) **The use of specified TCF target genes to identify drugs for the treatment of cancer, in particular colorectal cancer, in which TCF/beta-catenin/WNT signalling plays a central role**

(71) Applicant: Kylix B.V., 3971 JD Driebergen (NL)
(72) Inventor: Clevers, Johannes Carolus, 3712 AP Huis Ter Heide (NL); Gomez, Eduard Battle, 3582 JD Utrecht (NL); van de Wetering, Marcus Lambertus, 3994 TD Houten (NL); Sancho Suils, Elena, 3582 JD Utrecht (NL)
(74) Representative: Van Someren, Petronella F. H. M.

(57) **Abstract**

The present invention relates to the use of inhibitors of the expressed proteins of TCF target genes whose expression is regulated by TCF/β-catenin complexes for the preparation of a therapeutical composition for the treatment of cancers in which TCF/β-catenin signaling is deregulated. Such inhibitors can be antibodies, small molecules, antisense RNA and dsRNA for use in RNAi. The invention also relates to these inhibitors *per se*.

## Description

The present invention relates to the use of genes whose expression is regulated by TCF/β-catenin complexes in colon carcinoma cells for the identification and development of small molecule inhibitors, antibodies, antisense molecules, RNA interference (RNAi) molecules and gene therapies against these target genes and/or their expression product for the treatment of cancer in which deregulated TCF/β-catenin signalling occurs, in particular colorectal cancer and melanomas. In addition the invention relates to a method for the development of the small molecule inhibitors and antibodies. The invention also relates to the small molecule inhibitors, antibodies, antisense molecules, RNAi molecules and therapeutic genes per se and to their use in the treatment and diagnosis of cancer in which deregulated TCF/β-catenin/WNT signalling occurs and to pharmaceutical compositions comprising them.

The colorectal mucosa contains large numbers of invaginations known as the crypts of Lieberkühn. Epithelial cells in these structures are constantly renewed in a coordinated series of events comprising proliferation, cell migration and differentiation along the crypt axis towards the intestinal lumen. Pluripotent stem cells are believed to reside at the bottom positions of the crypt. From these stem cells, progenitors are generated that occupy the lower third of the crypt, the amplification compartment. Cells in this compartment divide approximately every 12 hours until their migration brings them to the mid-crypt region. Here, they cease proliferating and differentiate into one of the functional cell types of the colon. At the surface epithelium, cells undergo apoptosis and/or extrusion into the lumen. The complete process takes approximately 3-5 days.

Colorectal cancer (CRC) is one of the most common malignancies in the western world. The transition of an intestinal epithelial cell into a fully transformed, metastatic cancer cell is a slow process, requiring the accumulation of mutations in multiple proto-oncogenes and tumour suppressor genes. The APC gene, originally cloned from patients with the rare genetic disorder Familial Adenomatous Polyposis, is mutated in the vast majority of sporadic CRCs.

The APC protein resides in the so-called destruction complex, together with GSK3β, axin/conductin and β-catenin. In this complex, phosphorylation by GSK3β targets β-catenin for ubiquitination and destruction by the proteasome. Signalling by the extracellular factor WNT inhibits GSK3β activity. As a result, β-catenin accumulates in the nucleus where it binds members of the TCF family and converts these WNT effectors from transcriptional repressors into transcriptional activators. The terms "TCF/β-catenin signalling" and "WNT-signalling" are commonly used to describe the same signalling pathway.

In cancer, truncating mutations in *APC* and axin/conductin, as well as mutations in the GSK3β-target residues in β-catenin all lead to the formation of constitutive nuclear β-catenin/TCF complexes. Activating mutations of the WNT pathway are the only known genetic alterations present in early premalignant lesions in the intestine, such as aberrant crypt foci and small polyps. Thus, these mutations appear to initiate the transformation of colorectal epithelial cells.

In the intestinal epithelium, TCF4 is the most prominently expressed TCF family member. Gene disruption in the murine germ line has revealed that during embryonic development TCF4 is required to establish the proliferative progenitors of the prospective crypts in the small intestine.

To better understand the contribution of constitutive β-catenin/TCF activity to the colorectal transformation process, the inventors have undertaken a large-scale analysis of the downstream genetic program activated by β-catenin/TCF in CRC cells.

During this research it was found that inhibition of β-catenin/TCF activity in fully malignant colorectal cancer cells causes these cells to arrest in G1. DNA array analysis revealed the downregulation of a small set of transcripts. These genes were expressed in polyps, but also in the normal proliferative compartment of colon crypts. Accordingly, the presence of nuclear β-catenin in this compartment was demonstrated, suggesting that WNT signaling is controlling the self-renewing amplification compartment in the adult intestine. In addition, the induction of multiple marker genes of intestinal differentiation upon inhibition of β-catenin/TCF in CRC cells was observed. It was also found that the cell cycle inhibitor p21^{CIP1/WAF1} is an important mediator of this effect. It was concluded that β-catenin/TCF inhibits differentiation and imposes a crypt progenitor-like phenotype on CRC cells.

Moreover, disruption of β-catenin/TCF-activity in CRC cells restores the physiological program of epithelial differentiation, despite the presence of multiple other mutations present in these cells.

Thus, a group of target genes was identified whose expression is regulated by TCF/β-catenin complexes. In colon carcinoma cells TCF/β-catenin signalling is deregulated and the resulting inappropriate expression of these target genes is considered to promote carcinogenesis. The transactivation of TCF target genes induced by mutations in APC or β-catenin is believed to represent the primary transforming event in colorectal cancer.

The identification of the target genes of the TCF/β-catenin signalling pathway provides the opportunity to develop therapeutical compounds or therapies that restore or neutralize the inappropriate expression of these genes when TCF/β-catenin signalling is deregulated. By normalizing the expression pattern of one or more of the target gene the drugs can halt or reverse the further development of existing cancer cells, such as colon carcinoma cells, for example by the induction of differentiation of the cancer cells thus restoring the normal cycle of events.

The interference in the inappropriate expression of the target genes can be achieved via the expressed proteins and/or via the transcripts of the genes. These two ways require different active molecules as will be explained herein below.

The present invention relates according to a first aspect thereof to the use of these target genes or their expression products for the development of therapeutical compounds, in particular antibodies, small molecules, antisense molecules and RNAi molecules, and gene therapies for treating cancers in which TCF/β-catenin/WNT signalling is deregulated, in particular colorectal cancer and melanomas.

This is achieved in a first embodiment by characterizing the expression product of the target gene and the production of antibodies against the expressed protein or of small molecules that bind the expressed protein in a way that inhibits or abrogates its biological function.

According to a second embodiment, the target gene sequence information is used to design antisense molecules, RNAi molecules or gene therapies.

A further aspect of the present invention relates to the use of the target genes or their products for the development of reagents for diagnosis of cancers in which the TCF/β-catenin/WNT signalling is deregulated.

The target genes that were identified according to the invention are the following: CD44, c-Kit, G protein-coupled receptor 49 (GPR49), Solute Carrier Family 12 member 2 (SLC12A2), Solute Carrier Family 7 member 5, Claudin 1(CLDN), SSTK serine threonine kinase, FYN oncogene, EPHB2 receptor tyrosine kinase, EPHB3 receptor tyrosine kinase, c-ETS2, c-Myc, c-Myb, ID3, POLE3, Bone Morphogenetic Protein 4 (BMP4), Kit ligand (KITLG), GPX2, GNG2, CDCA7, ENC1, HSPC156, the gene represented by IMAGE clone 1048671, the gene represented by IMAGE clone 1871074, the gene identified with Celera ID hCG27486, the gene represented by IMAGE clone 294873, the gene identified with Celera ID hCG38927, the gene represented by IMAGE clone 742837, the gene represented by IMAGE clone 753028, the gene identified with Celera ID hCG37727, the gene represented by IMAGE clone 294133, and the gene identified with Celera ID hCG1811066. Table I gives an overview of these target genes.

Based on the above TCF/β-catenin target genes novel therapeutic compounds and therapies are developed for the treatment of cancer, in particular colorectal cancer. Such therapeutic compounds are preferably antibodies, small molecule inhibitors, antisense molecules or RNAi molecules. In addition gene therapies are provided.

Such gene therapies are based on the generation of dominant-negative forms of target genes, which inhibit the function of their wild-type counterparts following their directed expression in a cancer cell. Promoters for use in gene therapy that are specifically activated by TCF/β-catenin to drive specific expression of dominant-negative or suicide genes in cancer cells with active TCF / β-catenin signalling are known from e.g. Lipinski et al., Mol Ther 2001 4:365 - High level β-catenin/TCF dependent transgene expression in secondary colorectal cancer tissue ; Chen & McCormick, Cancer Res. 2001 61:4445 - Selective targeting to the hyperactive β-catenin/TCF factor pathway in colon cancer cells ; Fuerer & Iggo, Gene Ther. 2002 9:270 - Adenoviruses with TCF-binding sites in multiple early promoters show enhanced selectivity for tumor cells with constitutive activation of the Wnt signalling pathway.

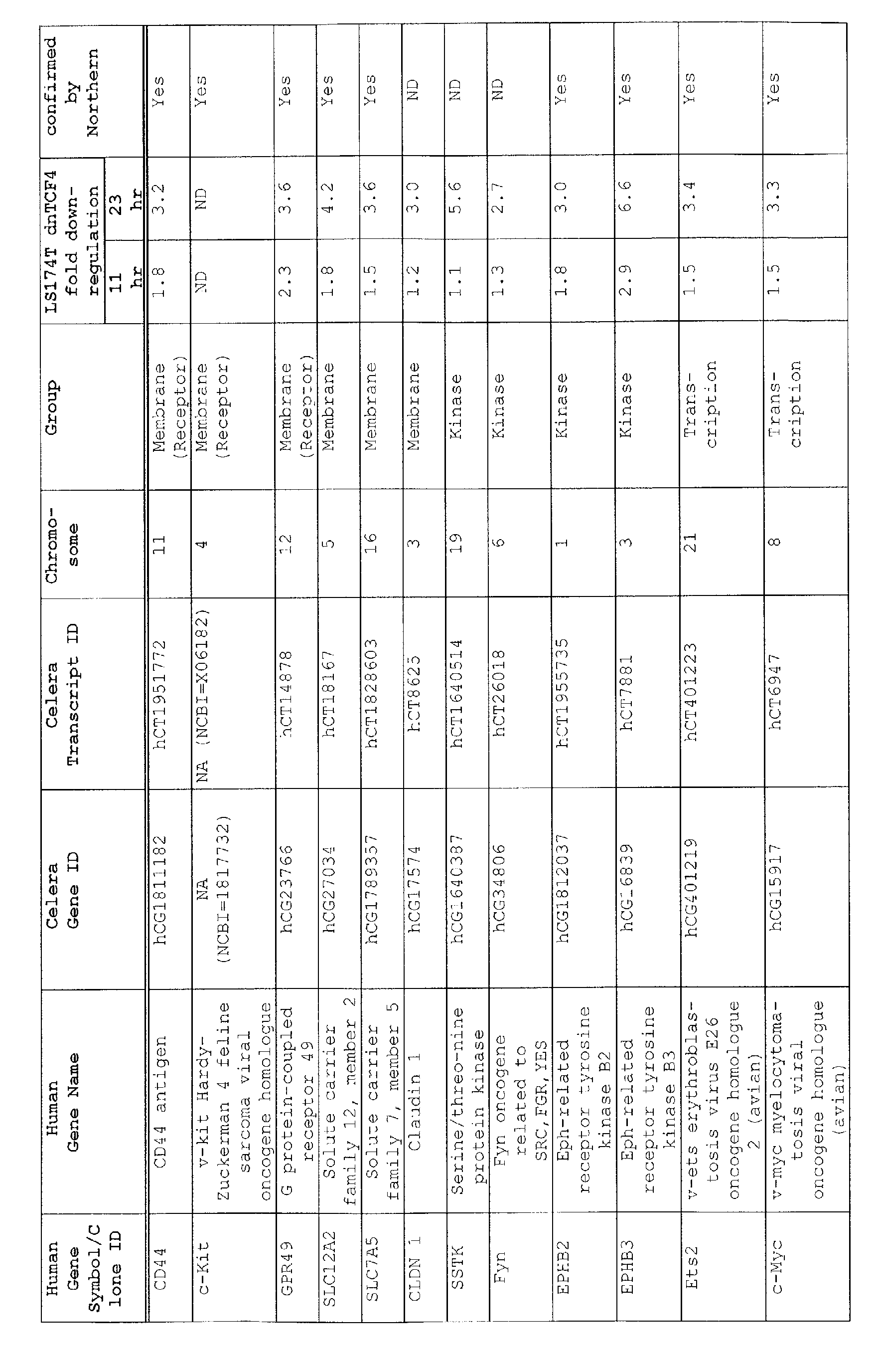

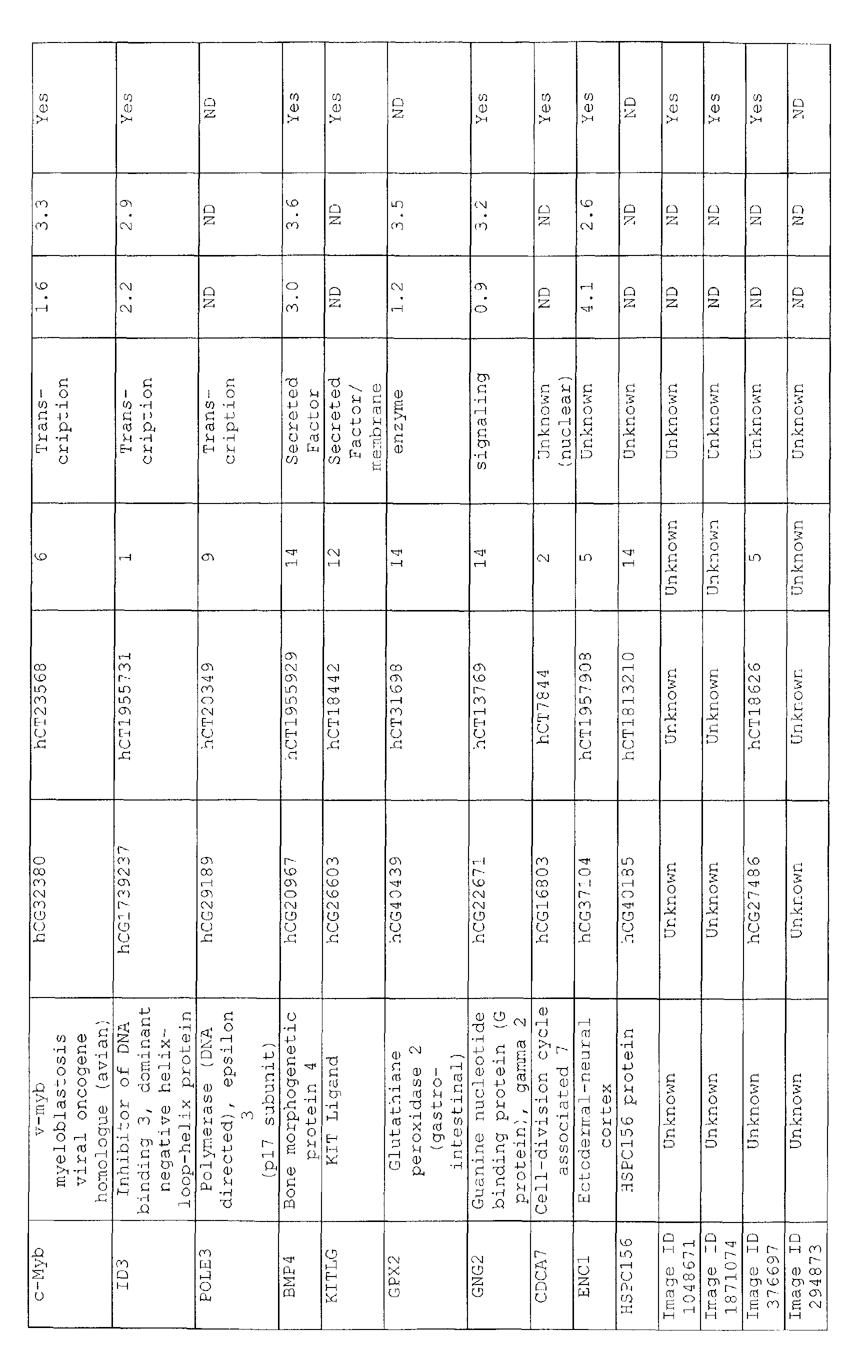

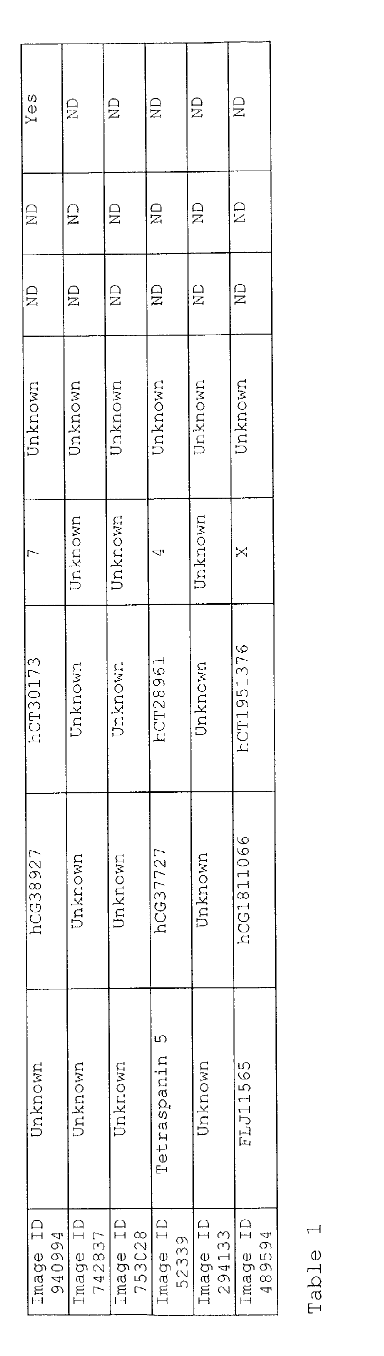

A method for the development of such therapeutic compounds comprises the steps:
a) identification of genes regulated by TCF/β-catenin in colon carcinoma cells, in particular by using microarray technologies;
b) validation of one or more of the identified genes as potential target gene(s) for the therapeutic compound by one or more of the following methods:
   - confirmation of the identified gene by Northern Blot analysis in colon carcinoma cell-lines;
   - determination of the expression profile of the identified gene in human colorectal tumors and normal tissue;
   - determination of the functional importance of the identified target genes for colorectal cancer;
c) production of the expression product of the target gene; and
d) use of the expression product of the target gene for the production or design of a therapeutic compound.

Once the target gene is validated and the expression product of the gene (the expressed protein) is produced there are various ways for developing a therapeutic compound for treating colorectal cancer. In colorectal carcinoma cells the TCF/β-catenin regulated target genes identified according to the invention are over expressed upon constitutive TCF/β-catenin activity. The compounds of the invention should thus neutralize the biological activity of the proteins expressed by the target gene in order to reverse the carcinoma phenotype.

A known way of neutralizing proteins is by means of antibodies. The invention according to a first aspect thereof thus relates to antibodies directed against the expression products of the target genes listed in Table I for use in the treatment of colorectal cancer. The production and evaluation of antibodies and their derivatives, such as scFv, Fab, chimeric, bifunctional and other antibody-derived molecules are well within the reach of the skilled person. Therapeutic antibodies are useful against target gene expression products located on the cellular membrane.

A second aspect of the invention relates to so-called small molecules interfering with the biological activity of the protein expressed by the target gene for use in the treatment of colorectal cancer. Small molecules are usually chemical entities that are developed on the basis of structure-function analysis of the protein with which they should interfere. Such analysis may involve determination of the crystal structure of the target protein. Based on the information thus obtained libraries of compounds can be screened or compounds may be designed and synthesized using medicinal and/or combinatorial chemistry. Alternatively, high throughput screening can be used to generate useful drug leads as well. After identification of a lead compound, this compound is screened for inhibition of target protein function using in-vitro and cell-based assays. After optimization of the lead compound with respect to its structure, toxicity profile and inhibition capability its efficacy as colon cancer therapeutic is tested in-vivo using animal models (e.g. Xenograft, APC^{min} mouse).

According to a third aspect of the invention antisense molecules are provided. Antisense drugs are complementary strands of small segments of mRNA. To create antisense drugs, nucleotides are linked together in short chains called oligonucleotides. Each antisense drug binds to a specific sequence of nucleotides in its mRNA target to inhibit production of the protein encoded by the target mRNA. By acting at this earlier stage in the disease-causing process to prevent the production of a disease-causing protein, antisense drugs have the potential to provide greater therapeutic benefit than traditional drugs which do not act until the disease-causing protein has already been produced. The invention relates to antisense molecules directed against the target genes listed in Table 1.

A further aspect of the invention relates to RNA interference (RNAi) molecules. RNAi refers to the introduction of homologous double stranded RNA to specifically target a gene's product, resulting in null or hypomorphic phenotype. RNA interference (RNAi) requires an initiation step and an effector step. In the first step, input double-stranded (ds) RNA is processed into 21-23-nucleotide 'guide sequences'. They may be single- or double-stranded. The guide RNAs are incorporated into a nuclease complex, called the RNA-induced silencing complex (RISC), which acts in the second effector step to destroy mRNAs that are recognized by the guide RNAs through base-pairing interactions. RNAi molecules are thus double stranded RNAs (dsRNAs) that are very potent in silencing the expression of the target gene. Potentially, a single dsRNA molecule could mark hundreds of mRNAs for destruction.

The invention relates further to gene therapy, in which the target genes are used for the design of dominant-negative genes which inhibit the function of the corresponding target gene following their specific expression in a cancer cell. Alternatively, RNAi approaches can be used gene therapeutically, for example by introducing a dsRNA producing sequence into a cancer cell.

The invention further relates to pharmaceutical compositions for treating cancers in which TCF/β-catenin signalling is deregulated, in particular colorectal cancer and melanomas, comprising a suitable excipient, carrier or diluent and one or more inhibitors of the proteins expressed by the TCF/b-catenin target genes or inhibitors of the mRNAs of the target genes.

The invention also provides diagnostic compositions for diagnosing cancer, in particular colorectal cancer and melanomas, comprising histological examination of tissues specimens using specific antibodies directed against TCF/β-catenin target gene products and/or *in situ* hybridisation analysis of TCF/β-catenin target gene expression using specific RNA probes directed against TCF/β-catenin target genes.

The present invention will be further illustrated in the Examples that follow and that are nonlimiting. In the Examples reference is made to the following figures:

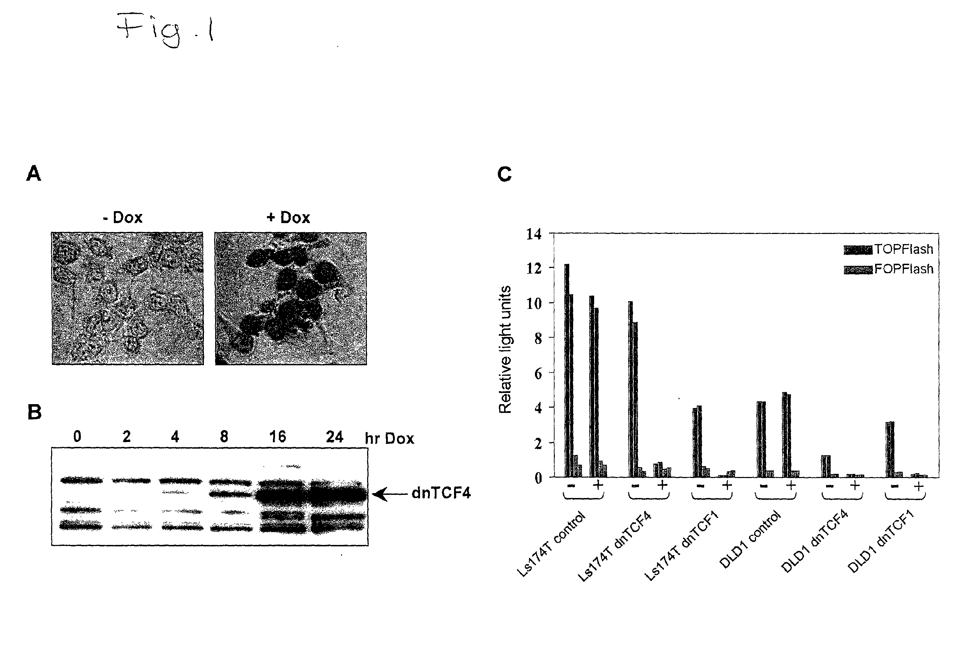

**Figure 1.** TCF/β-catenin driven transactivation is abrogated upon induction of dnTCF.
   **(A)** Inducible dnTCF4 expression in the CRC line Ls174T. Cells were stained for dnTCF4, 24 hours after induction with doxycycline. DnTCF4 is highly expressed in the nucleus.
   **(B)** dnTCF4 protein is induced as early as 4 hours after induction with doxycycline as analysed by western blot.
   **(C)** Both dnTCF1 and dnTCF4 abrogate β-catenin/TCF driven transcription in the β-catenin-mutant Ls174T as well as in the APC-mutant DLD1 cells. Activity of the TCF-reporter TOPFlash (purple bars) and control FOPFlash (green bars) after 24 hours with or without doxycycline (dox) is shown. Parental cell lines expressing the Tet-repressor alone are used as controls.
**Figure 2.** Northern blot analysis of genes regulated by β-catenin/TCF activity.
   **(A)** Representative examples of several target genes in Ls174T and DLD-1 transfectants. The indicated mRNAs were downregulated upon 24 hours of induction with doxycycline. The bottom panel shows the 28S ribosomal RNA as a loading control.
**Figure 3.** Expression of dnTCF induces G1 arrest.
   **(A)** Ls174T and DLD1 show a dramatic reduction in S phase cells upon dnTCF expression. The scatter profile of cells in G1 (blue), S (green) and G2/M (red) after 20 hours with or without doxycycline is shown. Numbers refer to the percentage of cells in S phase for each cell line analyzed. The results are representative of several independent experiments.
   **(B)** Proliferation was halted in Ls174T and DLD1 transfectants. This was visualized by crystal violet staining of cell cultures after 5 days of dnTCF expression.

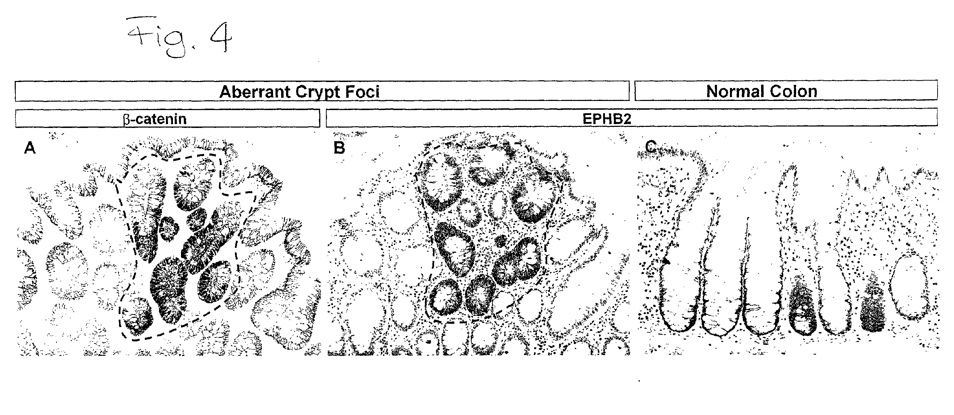

**Figure 4. (A-B)** The expression of nuclear β-catenin (A) perfectly correlates with that of EphB2 tyrosine kinase receptor (B) in aberrant crypt foci (ACF). Stainings were performed on serial sections of early human lesions. The dashed lines delimit the same ACF in both stainings. EphB2 is expressed at the bottom of the crypts (C).

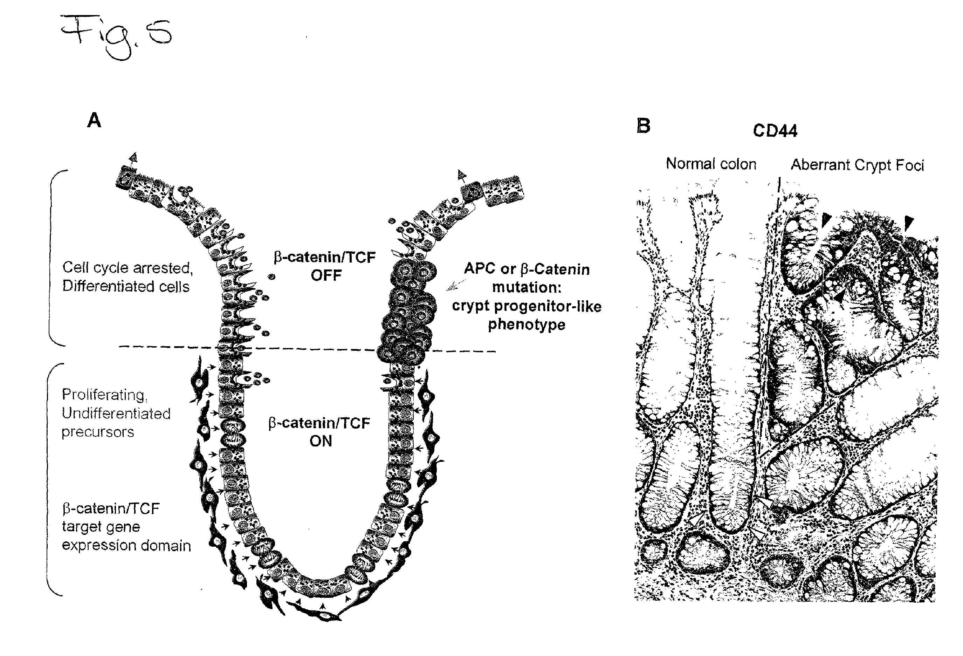

**Figure 5.** Model for the role of β-catenin/TCF in the early stages of intestinal tumorigenesis.
   **(A)** Schematic representation of a colon crypt and proposed model for polyp formation. At the bottom third of the crypt, the progenitor proliferating cells accumulate nuclear β-catenin. Consequently they express β-catenin/TCF target genes. An uncharacterised source of WNT factors likely resides in the mesenchymal cells surrounding the bottom of the crypt, depicted in red. As the cells reach the mid-crypt region, β-catenin/TCF activity is downregulated and this results in cell cycle arrest and differentiation. Cells undergoing mutation in *APC* or β-ca*tenin* become independent of the physiological signals controlling β-catenin/TCF activity. As a consequence, they continue to behave as crypt progenitor cells in the surface epithelium giving rise to ACFs.
   **(B)** CD44, a β-catenin/TCF target, exemplifies this model. It is expressed in the normal proliferative compartment at the bottom of the crypts (white arrowheads) and also in the early lesions arising at the surface epithelium (black arrowheads).

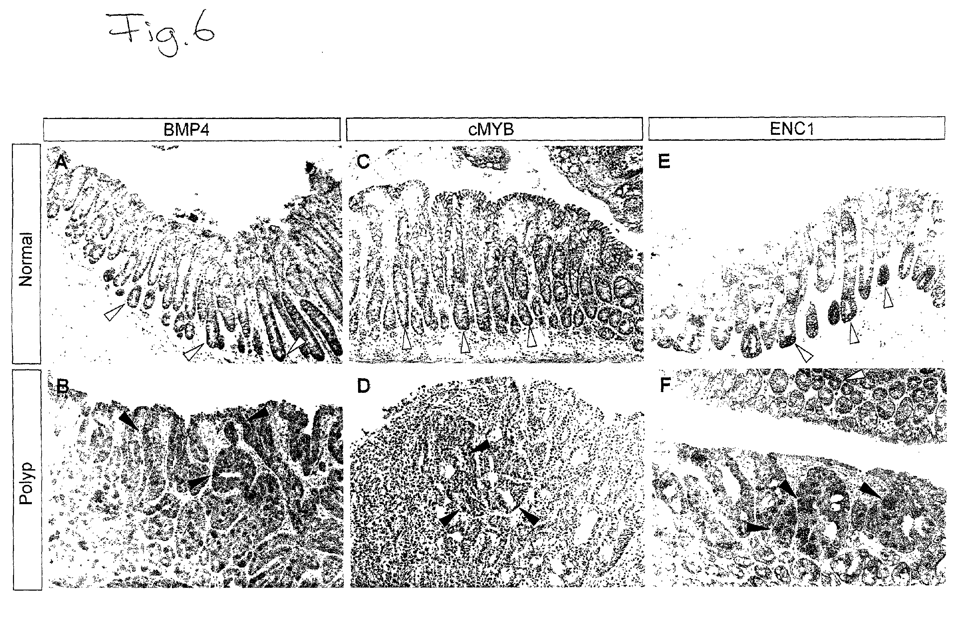

**Figure 6.** Expression of β-catenin/TCF target genes in normal colon and colorectal polyps.
   (A-F) Immunohistochemical analysis of the expression of Bmp4 (A and B), cMyb, © and D) and Enc1 (E and F) in normal mouse colon (A, C and E) or colorectal polyps from *min* mice (B, D and F). Target genes are highly expressed at the bottom of the normal crypts (white arrowheads) and in colorectal polyps arising at the surface epithelium (black arrowheads).

### EXAMPLES

### EXAMPLE 1

### Identification of target genes

### MATERIAL AND METHODS

### 1. Cell culture and transfections

Cells were grown in RPMI supplemented with 10% FCS and antibiotics. T-REx system (Invitrogen) was used according to manufacturer's instructions to generate inducible dnTCF or *p21*^{*CIP1*}/^{*WAF1*} inducible CRC cell lines. In short, 10⁷ cells were transfected by electroporation with 20 µg FspI linearized pcDNA₆TR . After 3 weeks of selection, blasticidin (10 µg/ml) resistant colonies were expanded and transfected with pcDNA₄TO-Luciferase. From each cell line, two clones showing the strongest induction were chosen. These were subsequently transfected with 20 µg PvuI linearized dnTC1 or dnTCF4 in pCDNA₄TO. After selection on Zeocin (500µg/ml), resistant colonies were tested for dnTCF induction by immunocytochemical staining after addition of doxycycline and selected for further studies. Ls174T/dnTCF4 constitutively expressing *c-MYC* and *c-MYB, C-ETS2, BMP4* and *ENC1* were derived in a similar manner by further transfecting these cells with pCDNA3 vectors (Invitrogen) carrying the appropriate cDNA and selecting for neomycin resistance.

### 2. Cell cycle analysis

3 × 10⁶ (Ls174T) or 10⁶ (DLD1) cells were seeded in 9 cm dishes and doxycycline was added (1µg/ml). After 20 hrs, BrdU (Roche) was added for 20 min. Cells were then collected and fixed in ethanol 70%. Nuclei were isolated, incubated with α-BrdU-FITC (BD) and cell cycle profiles were determined by FACS analysis. Crystal violet staining on methanol fixed cells were done on cells after 5 days in culture with or without the addition of doxycycline.

### 3. RNA isolation and northern analysis

RNA was isolated using Trizol (Gibco). Northern blots were performed according to standard procedures (Sambrook et al., 1989). Probes were obtained by appropriate restriction enzyme digestion of corresponding IMAGE clones (IMAGE consortium) spotted in the array.

### 4. Immunohistochemistry

The antibodies used in this study were obtained from the following sources: EPHB2 and EPHB3 from R&D systems; BMP4 from Novacastra; ENC1 from Pharmingen; c-MYB from Santa Cruz Biotechnology; p21^{CIP1/WAF1} from Pharmingen; carbonic anhydrase II from Rockland; β-catenin from Transduction Laboratories; TCF1 and TCF4 antibodies were described elsewhere (Barker et al., 1999; Castrop et al., 1995). Immunostainings were performed according to standard procedures. Briefly, sections were pretreated with peroxidase blocking buffer (100 mM Na-phosphate pH 5.8, 30 mM NaN₃, 0.2% H₂O₂) for 20 minutes at room temperature after dewaxing and hydration. Antigen retrieval was performed by boiling samples in 10 mM Na-citrate buffer pH 6.0, for 20 minutes. For β-catenin stainings, samples were boiled for 45 minutes in 40 mM Tris pH 8.0 containing 1 mM EDTA. Incubation of antibodies was performed in 1% BSA in PBS 1 hour at room temperature. In all cases, the Envision+ kit (DAKO) was used as a secondary reagent. Stainings were developed using DAB (brown precipitate). Slides were then counterstained with hematoxylin and mounted.

### 5. Probe preparation and microarray procedures

mRNA was extracted from cells using the Fasttrack 2.0 procedure (Invitrogen Inc.) following the manufacturer's directions. Fluorescent labeled cDNA was prepared from 1 µg of polyA mRNA by oligo dT-primed polymerization using Superscript II reverse transcriptase in the presence of either Cy3- or Cy5- labeled dCTP as described (website:
http://cmgm.stanford.edu/pbrown/protocols.html). The appropriate Cy3- and Cy5- labeled probes were pooled and hybridized to microarrays in a volume of 25 µl under a 22×14 mm glass coverslip for 16 hr. at 65°C and washed at a stringency of 0.2XSSC. The microarray contains 24,000 DNA spots representing approximately 10,000 known full-length cDNAs and 14,000 ESTs of clones made available by Research Genetics, which are listed in the supplementary information.

Fluorescent images of hybridized microarrays were obtained using a genepix 4000 microarray scanner (Axon Instruments, Inc). Images were analyzed with scanalyze (M. Eisen; http:/ / WWW. microarrays. org / software) or with genepix 3.0. Fluorescence ratios were stored in a custom database. Fluorescent ratios were calibrated independently for each array by applying a single scaling factor to all fluorescent ratios from each array; this scaling factor was computed so that the median fluorescence ratio of the measured spots on each array was 1.0. We selected genes represented by good-quality spots for which the fluorescent intensity in each channel was greater than 1.5 times the local background.

### RESULTS

### 1. Generation and characterization of inducible dnTCF cell lines

To determine the role of β-catenin/TCF complexes in established CRC cells, cell lines were constructed carrying doxycycline-inducible expression plasmids encoding N-terminally truncated versions of TCF factors. Such dominant-negative TCF (dnTCF) proteins do not bind β-catenin and therefore act as potent inhibitors of the endogenous β-catenin/TCF complexes present in CRC. As the recipient cell line, the CRC cell line Ls174T, which expresses mutant β-catenin protein, yet is diploid and carries wild-type alleles of *p53* and *APC* was initially chosen. Multiple clones were isolated and tested for inducibility of dnTCF4 expression.

Strong nuclear dnTCF4 staining was observed after doxycycline (Dox) induction of positive transfectants (Figure 1A). Accumulation of the induced protein could be detected as early as 4 hours after the addition of doxycycline (Figure 1B). CRC cell lines such as Ls174T that carry WNT pathway mutations constitutively activate TCF reporters (pTOPFlash). Several clones were selected in which the inducible expression of dnTCF4 completely abrogated this constitutive pTOPFlash activity (Figure 1C). Induction of dnTCF4 in such clones imposed a robust cell cycle block (see below), but did not result in the onset of apoptosis.

### 2. The genetic program under the transcriptional control of β-catenin/TCF activity in CRC cells

The spectrum of target genes controlled by β-catenin/TCF in CRC cells was expected to hold the key to understanding the primary transformation of intestinal cells. By DNA array analysis, it was asked which genes were specifically affected in their expression upon the induction of dnTCF4. mRNA was isolated at 11 hours and 23 hours after the initiation of the experiment with or without the addition of doxycycline. cDNA prepared from the uninduced samples was labeled with Cy3, while the induced cDNA samples were labeled with Cy5. At each time point, the uninduced and induced cDNA samples were mixed and hybridized in duplicate to a DNA array consisting of approximately 24,000 cDNA spots representing known genes or EST clusters. Fluorescent images were analysed as detailed in experimental procedures.

A single criterium was applied to the array data set: i.e. a decrease of at least 2.5 fold in both measurements at the 23 hour time point. This defined a small set of 35 entries that were downregulated when we abrogated β-catenin/TCF activity in Ls174T cells expressing dnTCF-4 (Table I).

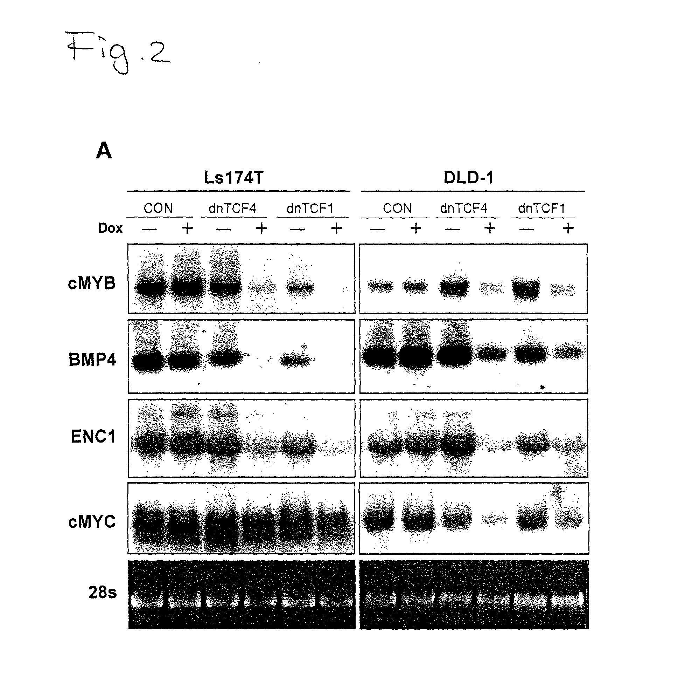

For a number of downstream genes defined in the Ls174T cells expressing dnTCF4, northern blot analysis was performed before and after induction of dnTCF4. This invariably confirmed the DNA array data (Table I and Figure 2A). The down-regulation of the reported TCF4 target gene *c-MYC* did not meet the 2.5 fold selection criterion, decreasing by an average 1.8 fold. However, its relatively modest, but consistent down-regulation was confirmed by northern blot (Figure 2A).

To further investigate the effects of β-catenin/TCF inhibition, Ls174T cells expressing dnTCF1, the natural dominant-negative isoform of TCF1 expressed in the intestinal epithelium were constructed. Likewise, DLD-1 cells, a CRC cell line with wild type β-catenin but mutated *APC* and *p53,* was engineered to express inducible dnTCF1 or dnTCF4.

Again, doxycycline-induced expression of dnTCFs in all cell lines resulted in the abrogation of pTOPflash activity (Figure 1C) and cell cycle arrest (see below). Northern blot analysis was performed in the above dnTCF expressing cell lines. Almost invariably, the target genes listed in Table I were also strongly downregulated by dnTCF1 in Ls174T (Figure 2A). In addition, the DLD-1 cells showed a similar pattern of target gene expression upon inhibition of β-catenin/TCF activity by dnTCF1 or dnTCF4 (Figure 2A).

### 3. Inhibition of β-catenin/TCF activity leads to cell cycle arrest and differentiation in CRC cells.

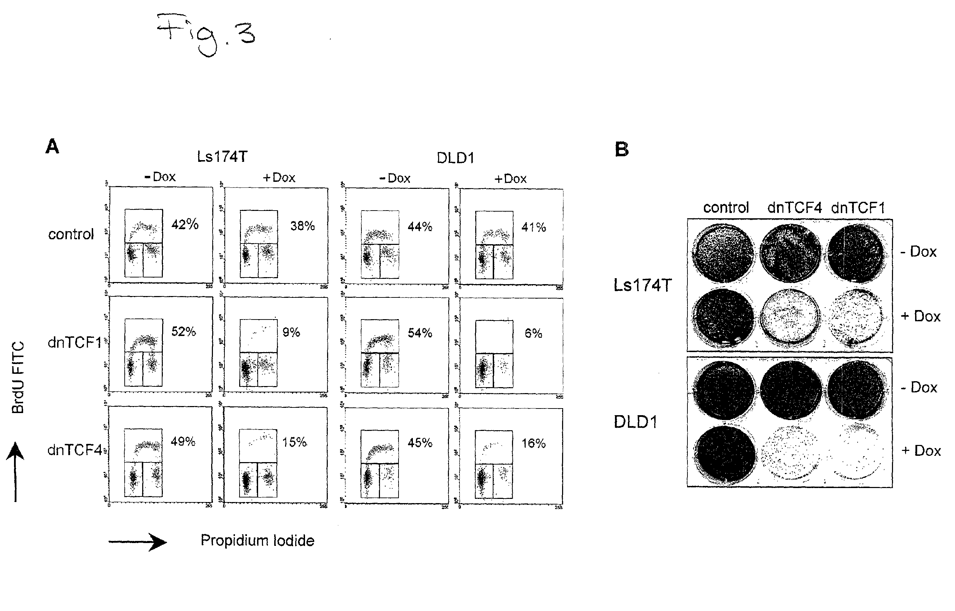

The induction of dnTCF4 or dnTCF1 in both Ls174T and DLD1 cell lines had a dramatic effect on cell cycling. Within 20 hours, a robust G1 arrest was induced (Figure 3A). Accordingly, cell proliferation was halted upon doxycycline induction of dnTCFs as visualized by crystal violet staining of cell cultures induced for 5 days (Figure 3B).

### 4. The genetic program controlled by β-catenin/TCF in CRC cells is physiologically active in colonic epithelium

In order to validate the β-catenin/TCF target genes described in this example, immunohistochemical analyses of those entries for which antibodies were available were performed on early intestinal neoplastic lesions. In Figure 4, a representative example of this analysis is shown. As expected, a strict correlation between the accumulation of nuclear β-catenin (Figure 4A) and the expression of EPHB2 (Figure 4B) was observed in early colorectal lesions. Many other downregulated genes listed in Table I were overexpressed in early intestinal polyps from *Min* mice or in aberrant crypt foci (ACF) from FAP patients (Figure 5, Figure 6).

More strikingly, it was found that EPHB2 was not only expressed in polyps, but also in cells within the proliferative compartment at the bottom of normal colon crypts (Figure 4C). This pattern was invariably confirmed for all target genes tested by immunohistochemistry. These included *c-MYB, BMP4*, *ENC1,* (Figure 6), *EPHB3* (not shown), and *CD44* (Figure 5B).

Thus, the observed gene expression changes in CRC cells recapitulated the physiological differentiation of crypt progenitor cells during their migration towards the luminal surface of the intestine.

### DISCUSSION

The data presented here provide a view of the genetic program driven by β-Catenin/TCF activity in CRC cells. The expression of a surprisingly limited set of genes is dependent on the presence of active β-catenin/TCF complexes.

A hallmark of cancer is deregulated proliferation. Abrogation of β-catenin/TCF activity in all CRC cell lines tested here induced a robust arrest in the G1 phase of the cell cycle, demonstrating that the activity of the β-catenin/TCF complex represents the major force driving cell proliferation in intestinal cells.

β-Catenin and TCF modulate cell cycle control by activating genes that promote cell cycling (e.g. c-myc), but also by repressing cell cycle inhibitors (p21^{CIP1/WAF1} ; results not shown). β-Catenin/TCF represents the main upstream regulator of the cell cycle machinery in epithelial intestinal cells.

In conclusion, the above observations demonstrate that TCF constitutes the dominant switch between the proliferating progenitor and the differentiated intestinal cell. This is recapitulated in the CRC cells used in this study, despite the presence of multiple additional mutations in these cells. This example validates that the genetic program controlled by TCF/β-catenin signaling can be used as the basis for the development of a therapeutic strategy to revert the transformed phenotype in colorectal cancer.

### EXAMPLE 2

### Development of drugs for the treatment of colorectal cancer on the basis of the target genes defined in example 1

### 1. Identification and validation of the target genes

Example 1 demonstrates the identification of Target Genes represented on cDNA/oligonucleotide microarrays which are regulated by TCF/β-catenin transcription factor complexes. Subsequently, the regulated expression of target genes in colon cancer cell-lines is confirmed via Northern blot analysis using gene specific probes as described in Example 1.

In order to confirm that the expression of the target genes that were found in Example 1 are linked to the TCF/β-catenin complex, target gene expression is also evaluated in tissues known to have active TCF/β-catenin complexes (for example, intestinal epithelium and colorectal polyps) using gene-specific antibodies, *in situ* hybridization with gene-specific probes and/or RT-PCR with gene-specific primers.

After that, the expression profile of the target gene in human/mouse cell-lines and tissues is determined via Northern blot analysis and/or RT-PCR. This is done because ubiquitous expression of the target gene may be indicative of possible side-effects of therapeutics designed to block the target gene's function in-vivo.

### 2. Obtaining the complete gene

The identification of the target genes on a microarray does not identify the complete gene. The next step in the development of a therapeutic compound is therefore generation of full-length clones for the EST sequences shown to be regulated via TCF/β-catenin in the colon carcinoma cell-lines. This is achieved by searching databases for full-length EST clones and/or techniques such as RT-PCR, RACE and hybridization screening of cDNA libraries.

### 3. Identification of binding sites within the target genes

Putative TCF binding sites [(A/T) (A/T) CAA (A/T) GG] within target gene promoters are identified according to Van de Wetering et al., (1991) Identification and cloning of TCF-1, a T-lymphocyte-specific transcription factor containing a sequence-specific HMG box EMBO J 11: 3039-3044). Enhancers are identified using web-based prediction programs such as Genomatix (www . genomatix. gsf. de/ promoterinspector). This provides an indication that a gene is regulated via direct binding of TCF/β-catenin complexes. However, many binding sites will not be identified due to the vast tracts of genomic DNA containing the target gene which may harbor distant enhancers. Testing of functionality of putative TCF binding sites in target genes is then performed via mutational analysis. Promoter regions of target genes containing the original or mutated putative TCF binding sites are cloned upstream of TK-Luciferase reporter genes cassettes and analysed for their ability to drive expression of the reporter gene in the presence of TCF/β-catenin complexes in cultured cell-lines, such as described in Tetsu and McCormick, (1999) (β-catenin regulates expression of cyclin D1 in colon carcinoma cells. Nature 398: 422-426)). A correlation between mutation of a TCF binding site and loss of reporter gene expression indicates that direct binding of TCF/β-catenin is contributing to target gene expression.
Determination of the ability of ectopic target gene expression to overcome defects in the growth of colon carcinoma cells caused by blocking TCF/β-catenin signaling is performed as in Example 1 to establish whether expression of a single target gene is sufficient to overcome the block in cell-cycle and differentiation of the colon cancer cell.

### 4. Confirmation of involvement of target genes in colon cancer

Subsequently it is important to establish the contribution of specific target genes to colon cancer. The techniques used to do this are dominant-negative approaches, i.e. expression of target genes carrying deletions/mutations which suppress the function of their endogenous counterparts in colon cancer cell-lines; antisense/RNAi approaches, i.e. introduction of double-stranded RNA oligonucleotides designed to block expression of a specific target gene into colon cancer cell-lines (as described in Elbashir et al., (2001). Duplexes of 21-nucleotide RNAs mediate RNA interference in cultured mammalian cells. Nature 411: 494-498) and genetic approaches, such as generation of mice deficient for target gene expression in intestinal tissues using a combination of standard loxP knockout technologies and intestine-specific Cre mouse strains, or generation of transgenic mice expressing dominant-negative target genes. These mice strains are crossed with APC^{min} mice to determine whether loss of target gene function in-vivo has any adverse effect on colorectal polyp formation (as for example described by Oshima et al., (1996) Suppression of Intestinal Polyposis in APC^{D716} Knockout mice by inhibition of cyclooxygenase 2 (COX-2) Cell 87: 803-809).

Using these approaches it is determined whether loss of function of a specific target gene has any adverse effect on colon cancer cell-lines and/or on polyp formation in-vivo and thus insight is gained into whether therapeutics designed to specifically inhibit the function of these target genes are likely to be effective in combating colon cancer in humans.

Furthermore, the genetic programs affected by inhibition of target gene function in colon carcinoma cells are evaluated using microarrays. Thus, the function of the target gene in colon carcinoma cells is established and valuable information regarding the possible side-effects that inhibition of this gene function may have on genetic programs required for normal cell function is provided. By definition, many of the validated TCF/β-catenin target genes will be more highly expressed on colon carcinoma tissues than healthy tissues and some encode cancer-specific proteins, making these excellent targets against which to develop colon cancer therapeutics.

### 5. Identification or development of candidate compounds

### Antibodies

Validated target genes which express membrane-bound proteins are then selected as targets for conventional antibody-based therapies (for an example according to Schwartzberg (2001) Clinical experience with edrecolomoab: a monoclonal antibody therapy for colorectal carcinoma. Crit. Rev. Oncol. Hematol. 40: 17-24).

### Small molecules

Validated intracellular and membrane-expressed target proteins are furthermore selected as targets for developing small molecule compound-based therapies. For this their crystal structures are determined, either from published information available from web-based databases *(NCBI)* or using protein production and crystallization facilities. Structure analysis is performed with the computer programs SPOCK (Christopher J (1998). SPOCK, The structural properties observation and calculation kit), GRASP (Nicholls et al., (1991) Structure, Function and Genetics 11:281-283) and SWISS PDB Viewer (Guez and Peitsch (1997) SWISS-MODEL and the Swiss-Pdb Viewer: An environment for comparative protein modeling) or others.

In addition to the rational development of novel small molecules, high capacity screening of existing small molecule compound libraries generate lead compounds which become inhibitors of validated target proteins encoding enzymes such as protein kinases. Highly active inhibitors are co-crystallized with the enzyme and computer programs such as GOLD (Distributed via Cambridge Crystallographic Data Centre; Jones et al., (1995) J. Mol. Biol 245: 43-53) and CERIUS2/LUDI (Bohm (1992) The computer program Ludi: A new method for the de novo design of enzyme inhibitors. J. Comp. Aided Molec. Design 6:61-78) are used for structure-based design of improved inhibitors.

### Antisense molecules

These can be either antisense RNA or antisense oligodeoxynucleotides (antisense ODNs). They can be prepared synthetically or by means of recombinant DNA techniques. Both methods are well within the reach of the person skilled in the art. ODNs are smaller that complete antisense RNAs and have therefore the advantage that they can more easily enter the target cell. In order to avoid their digestion by DNAse ODNs, but also antisense RNAs are chemically modified. For targeting to the desired target cells the molecules are linked to ligands of receptors found on the target cells or to antibodies directed against molecules on the surface of the target cells.

### RNAi molecules

Double-stranded RNA corresponding to a particular gene is a powerful suppressant of that gene. The ability of dsRNA to suppress the expression of a gene corresponding to its own sequence is also called post-transcriptional gene silencing or PTGS. The only RNA molecules normally found in the cytoplasm of a cell are molecules of single-stranded mRNA. If the cell finds molecules of double-stranded RNA, dsRNA, it uses an enzyme to cut them into fragments containing 21-25 base pairs (about 2 turns of a double helix). The two strands of each fragment then separate enough to expose the antisense strand so that it can bind to the complementary sense sequence on a molecule of mRNA. This triggers cutting the mRNA in that region thus destroying its ability to be translated into a polypeptide. Introducing dsRNA corresponding to a particular gene will knock out the cell's own expression of that gene. This can be done in particular tissues at a chosen time. A possible disadvantage of simply introducing dsRNA fragments into a cell is that gene expression is only temporarily reduced. However, introducing into the cells a DNA vector that can continuously synthesize a dsRNA corresponding to the gene to be suppressed can provide a more permanent solution. RNAi molecules are prepared by methods well known to the person skilled in the art.

### Other compounds

To predict the location of critical contact sites for cofactors, ligands or other molecules contributing to the function of target proteins use is made of computer-based modeling with the programs mentioned above. Confirmation of essential contact sites in target proteins is performed by mutational analysis and subsequent identification of hydrophobic pockets located on the protein surface in the vicinity of these contact sites.

Computer modeling of "virtual" public compound libraries for binding to these hydrophobic pockets and testing of "best fit" compounds in in-vitro (ELISA) and in-vivo (cell-based) assays for inhibition of target protein function will allow determination of a structure-activity relationship for compound classes.

In addition, de-novo compound libraries are generated based on the information derived from the computer modeling described above using a combinatorial chemistry approach.

### 6. Evaluation of candidate compounds

Candidate compound are evaluated for cellular toxicity via commercially available service, such as MDS Pharma Services, USA. The evaluation of candidate compound efficacy in reducing polyp formation in APC^{min} mice according to Su et al., (1992) Multiple intestinal neoplasia caused by a mutation in the murine homologue of the APC gene. Science 256: 668-670. The candidate compounds are also tested in other models predictive for colorectal cancer (e.g. Xenograft).

## Claims

1. Use of inhibitors of the expressed proteins of TCF target genes whose expression is regulated by TCF/β-catenin complexes for the preparation of a therapeutical composition for the treatment of cancers in which TCF/β-catenin signaling is deregulated.

2. Use as claimed in claim 1, wherein the inhibitors are antibodies or derivatives thereof directed against the expression products of the target genes that are expressed on the cell membrane.

3. Use as claimed in claim 2, wherein the derivatives are selected from the group consisting of scFv fragments, Fab fragments, chimeric antibodies, bifunctional antibodies, and other antibody-derived molecules.

4. Use as claimed in claim 1, wherein the inhibitors are small molecules interfering with the biological activity of the protein expressed by the target gene.

5. Use of inhibitors of the mRNA transcripts of TCF target genes whose expression is regulated by TCF/β-catenin complexes for the preparation of a therapeutical composition for the treatment of cancers in which TCF/β-catenin signaling is deregulated.

6. Use as claimed in claim 5, wherein the inhibitors are antisense molecules, in particular antisense RNA or antisense oligodeoxynucleotides.

7. Use as claimed in claim 5, wherein the inhibitors are double stranded RNA molecules for RNA interference.

8. Use as claimed in claim 1, wherein the treatment comprises gene therapy.

9. Use as claimed in any one of the claims 1-8, wherein the therapeutical composition is for treatment of Familial Adenomatous Polyposis (FAP).

10. Use as claimed in any one of the claims 1-8, wherein the therapeutical composition is for treatment of colorectal cancer.

11. Use as claimed in any one of the claims 1-8, wherein the therapeutical composition is for treatment of melanomas.

12. Use of TCF target genes whose expression is regulated by TCF/β-catenin complexes for the diagnosis of cancers in which TCF/β-catenin signaling is deregulated.

13. Use as claimed in claim 12, wherein the diagnosis is performed by means of histological analysis of tissue specimens using specific antibodies directed against target gene products, and/or *in situ* hybridization analysis of TCF/β-catenin target gene expression levels in tissue specimens using specific RNA probes directed against TCF/β-catenin target gene sequences.

14. Use as claimed in any one of the claims 1-13, wherein the target gene is selected from the group consisting of CD44, c-Kit, G protein-coupled receptor 49 (GPR49), Solute Carrier Family 12 member 2 (SLC12A2), Solute Carrier Family 7 member 5, Claudin 1 (CLDN), SSTK serine threonine kinase, FYN oncogene, EPHB2 receptor tyrosine kinase, EPHB3 receptor tyrosine kinase, c-ETS2, c-Myc, c-Myb, ID3, POLE3, Bone Morphogenetic Protein 4 (BMP4), Kit ligand (KITLG), GPX2, GNG2, CDCA7, ENC1, HSPC156, the gene represented by IMAGE clone 1048671, the gene represented by IMAGE clone 1871074, the gene identified with Celera ID hCG27486, the gene represented by IMAGE clone 294873, the gene identified with Celera ID hCG38927, the gene represented by IMAGE clone 742837, the gene represented by IMAGE clone 753028, the gene identified with Celera ID hCG37727, the gene represented by IMAGE clone 294133, and the gene identified with Celera ID hCG1811066.

15. Inhibitor compound directed against the expressed proteins of a TCF target gene the expression of which is regulated by TCF/β-catenin complexes for use in the treatment of colorectal cancer.

16. Inhibitor compound as claimed in claim 15, which is an antibody or derivatives thereof directed against the expression products of a target gene that is expressed on the cell membrane.

17. Inhibitor compound as claimed in claim 16, wherein the derivative is selected from the group consisting of scFv fragments, Fab fragments, chimeric antibodies, bifunctional antibodies, or other antibody-derived molecules.

18. Inhibitor compound as claimed in claim 15, which is a small molecule interfering with the biological activity of the protein expressed by the target gene.

19. Inhibitor compound directed against the transcription product (mRNA) of a TCF target gene the expression of which is regulated by TCF/β-catenin complexes for use in the treatment of colorectal cancer.

20. Inhibitor compound as claimed in claim 19, which is an antisense molecule, in particular an antisense RNA or an antisense oligodeoxynucleotide.

21. Inhibitor compound as claimed in claim 19, which is a double stranded RNA molecule for RNA interference.

22. Inhibitor compound as claimed in any one of the claims 15-21, wherein the target gene is selected from the group consisting of CD44, c-Kit, G protein-coupled receptor 49 (GPR49), Solute Carrier Family 12 member 2 (SLC12A2), Solute Carrier Family 7 member 5, Claudin 1(CLDN), SSTK serine threonine kinase, FYN oncogene, EPHB2 receptor tyrosine kinase, EPHB3 receptor tyrosine kinase, c-ETS2, c-Myc, c-Myb, ID3, POLE3, Bone Morphogenetic Protein 4 (BMP4), Kit ligand (KITLG), GPX2, GNG2, CDCA7, ENC1, HSPC156, the gene represented by IMAGE clone 1048671, the gene represented by IMAGE clone 1871074, the gene identified with Celera ID hCG27486, the gene represented by IMAGE clone 294873, the gene identified with Celera ID hCG38927, the gene represented by IMAGE clone 742837, the gene represented by IMAGE clone 753028, the gene identified with Celera ID hCG37727, the gene represented by IMAGE clone 294133, and the gene identified with Celera ID hCG1811066.

23. Diagnostic agent for diagnosing cancers in which TCF/β-catenin signaling is deregulated.

24. Diagnostic agent as claimed in claim 23, which is a specific antibody directed against a TCF/β-catenin target gene or an RNA probe specific for a TCF/β-catenin target gene sequence.

25. Therapeutical composition for the treatment of cancers in which the TCF/β-catenin signaling is deregulated, comprising a suitable excipient, carrier or diluent and one or more inhibitor compounds as claimed in claims 15-22.

26. Diagnostic composition for the diagnosis of cancers in which the TCF/β-catenin signaling is deregulated, comprising a suitable excipient, carrier or diluent and one or more diagnostic compounds as claimed in claims 23-24.

27. Compositions as claimed in claim 25 or 26, wherein the cancer is colorectal cancer, melanoma or Familial Adenomatous Polyposis (FAP).

28. Method for the development of therapeutic inhibitor compounds as claimed in claims 15-22, which method comprises the steps:
a) identification of genes regulated by TCF/β-catenin in colon carcinoma cells, in particular by using microarray technologies;
b) validation of one or more of the identified genes as potential target gene(s) for the therapeutic compound by one or more of the following methods:
- confirmation of the identified gene by Northern Blot analysis in colon carcinoma cell-lines;
- determination of the expression profile of the identified gene in human colorectal tumors and normal tissue;
- determination of the functional importance of the identified target genes for colorectal cancer;
c) production of the expression product of the target gene; and
d) use of the expression product of the target gene for the production or design of a therapeutic compound.

29. Method as claimed in claim 28, wherein the target gene identified in step a) is selected from the group consisting of CD44, c-Kit, G protein-coupled receptor 49 (GPR49), Solute Carrier Family 12 member 2 (SLC12A2), Solute Carrier Family 7 member 5, Claudin 1(CLDN), SSTK serine threonine kinase, FYN oncogene, EPHB2 receptor tyrosine kinase, EPHB3 receptor tyrosine kinase, c-ETS2, c-Myc, c-Myb, ID3, POLE3, Bone Morphogenetic Protein 4 (BMP4), Kit ligand (KITLG), GPX2, GNG2, CDCA7, ENC1, HSPC156, the gene represented by IMAGE clone 1048671, the gene represented by IMAGE clone 1871074, the gene identified with Celera ID hCG27486, the gene represented by IMAGE clone 294873, the gene identified with Celera ID hCG38927, the gene represented by IMAGE clone 742837, the gene represented by IMAGE clone 753028, the gene identified with Celera ID hCG37727, the gene represented by IMAGE clone 294133, and the gene identified with Celera ID hCG1811066.
